# EUROPÄISCHE PATENTSCHRIFT

(11) **EP 1 458 334 B1**
(45) Veröffentlichungstag und Bekanntmachung des Hinweises auf die Patenterteilung: **22.03.2006**
(21) Anmeldenummer: 02796686.0
(22) Anmeldetag: 19.12.2002
(51) Int. Cl.: A61K 8/97, A61K 35/74, A61Q 19/00, A61Q 19/08

(54) **HEIL-UND PFLEGEMITTEL AUF SAUERTEIGBASIS SOWIE VERFAHREN ZU SEINER HERSTELLUNG**
YEAST-BASED CURATIVE AND CARE PRODUCT AND METHOD FOR MAKING SAME
PRODUIT CURATIF ET DE SOIN A BASE DE LEVAIN ET SON PROCEDE DE PRODUCTION

(30) Priorität: 21.12.2001 EP 01130634
(43) Veröffentlichungstag der Anmeldung: 22.09.2004
(73) Patentinhaber: Woresan GmbH, 30916 Isernhagen (DE)
(72) Erfinder: WOLF, Gabriele, 30916 Isernhagen (DE)
(74) Vertreter: von Kreisler Selting Werner
(86) Internationale Anmeldenummer: PCT/EP2002/014543
(87) Internationale Veröffentlichungsnummer: WO 2003/053376

(56) Entgegenhaltungen:
- WO-A-00/10395
- DE-A- 4 025 303
- DE-U- 29 923 627

## Beschreibung

Die Erfindung betrifft ein Verfahren zur Herstellung eines Heil- und Pflegemittels für die Haut auf Basis eines ausgesäuerten Sauerteiges, ein Heil- und Pflegemittel erhältlich nach diesem Verfahren, ein Kosmetikum enthaltend ein Heil- und Pflegemittel sowie die Verwendung des erfindungsgemäßen Heil- und Pflegemittels in einem Kosmetikum.

Aus der DE-A-38 02 840 2 ist bereits, ein Verfahren zur Herstellung eines Milchsäure und lebensfähige Milchsäurebakterien enthaltenden Gärproduktes durch Versäuern einer wässrigen Aufschlämmung von gebackener Brotmasse auf Sauerteigbasis in milchsaurem Medium bekannt geworden, wobei als Brotmasse das Backprodukt eines ausgesäuerten Brotteiges eingesetzt wird. Dieses Gärprodukt soll als u. a. als Hilfsmittel bei der Behandlung des ganzheitlichen Organismus von Mensch, Tier und Pflanze einsetzbar sein.

Aus der DE-A-38 46 186 ist es auch bereits bekannt geworden, aus dem Gärschlamm der Spontanversäuerung von Sauerteigbroten gewonnene Präparate z. B. als Packungszuschlagstoff zur Behandlung von chronisch entzündlichen Krankheiten der Haut einzusetzen.

Durch die DT-B-26 11 972 ist es bereits bekannt geworden, zum Herstellen eines Sauerteigs für die Bereitung von Broten und Backwaren aus Getreideschroten und/oder Mehlen in der den Vorteig bildenden Getreidemaische die Sauerteigbakterien bis zur Einstellung jeglicher bakteriologischer Stoffwechselaktivität auszusäuern.

In der EP-B-0 530 861 ist eine topische, antimikrobielle pharmazeutische Zusammensetzung offenbart, die ein Gemisch aus C₆- bis C₁₈-Fettsäuren umfasst.

DE-U-299 23 627 betrifft ein Heil- und Pflegemittel zur Förderung der Durchblutung, zur Lösung von Muskelverspannungen, zur Hautreinigung und zur Hautregenerierung, das auf Körperteile aufgetragen oder als Zusatz zu einem Wasserbad verwendet wird. Das Produkt ist dadurch gekennzeichnet, dass es von einem Fermentationsprodukt eines ausgesäuerten Sauerteiges gebildet ist.

In dieser Druckschrift wird nichts hinsichtlich einer Vorbehandlung des als Substrat verwendeten Roggenschrots erwähnt.

WO-A-00/10395 offenbart ein Verfahren zur Herstellung eines lebenden, flüssigen Sauerteigprodukts, das nach kalter Lagerung für die Dauer von 10 Wochen mit einer Plackbildungskapazität von mindestens 5 x 10⁸ CFU/g Mehl in der Trockner aufweist. Keinerlei Hinweise werden gegeben, dass dieses Produkt als Heil- und Pflegemittel geeignet ist. In diesem Dokument wird als Puffermittel Calciumkarbonat eingesetzt, damit der pH Wert der dort beschriebenen Mischung nicht zu tief absinkt.

Natürliche durch Fermentation gebildete Produkte, die mit der Haut in Kontakt gebracht werden besitzen ein Potential, pflegebedürftige Haut positiv zu beeinflussen.

Aufgabe der vorliegenden Erfindung war es, die positiven Einflüsse, die insbesondere aus der DE-U-299 23 627 bekannten Wirkungen zu verbessern.

Überraschenderweise sind Fermentationsprodukte von Roggenschrot oder Roggenmehlen mit heterofermentativen Milchsäurebakterien, die vor der Fermentation mit α-Amylase behandelt worden sind, geeignet, die gestellte Aufgabe zu lösen.

Gegenstand der vorliegenden Erfindung ist ein Verfahren zur Herstellung eines Heil- und Pflegemittels für die Haut aus Fermentationsprodukten auf Basis eines ausgesäuerten Sauerteiges, wobei
- 1.1: einem Roggenmehl oder Roggenschrot ein natürlicher enzymatischer Starter und Wasser zugegeben wird, die Mischung einem Erhitzungsprozess auf 65 -75°C innerhalb von 3-5 Stunden ausgesetzt wird, wobei durch enzymatische Reaktionen eine starke Maltosebildung einsetzt,
- 1.2.: eine weitere Zugabe von Roggenmehl oder Roggenschrot, Wasser sowie eines bakteriologischen Impfguts aus der Gruppe der heterofermentativen Milchsäurebakterien, erfolgt
- 1.3.: und der Ansatz bis zur Einstellung der Stoffwechseltätigkeit der Mikroorganismen ausgesäuert wird zu einem pH Wert <4.

Insbesondere wird als enzymatischer Starter α-Amylase eingesetzt.

In einer besonderen Ausgestaltung des erfindungsgemäßen Verfahrens werden vorzugsweise Lactobacillus DSM 6037 und/oder Lactobacillus DSM 6129 eingesetzt.

In einer Weiterbildung des erfindungsgemäßen Verfahrens wird dem Fermentationsprodukt ein zweites kohlehydrathaltiges Substrat und Wasser sowie ein bakteriologisches Impfgut aus der Gruppe der heterofermentativen Milchsäurebakterien zugegeben und die Mischung ausgesäuert,
- der letzte Verfahrensschritt ggf. ein oder mehrmals wiederholt wird, wobei die Mischung abschließend bis zur Einstellung jeglicher bakteriologischer Stoffwechselaktivität ausgesäuert wird und
- das Fermentationsprodukt einem stabilisierenden Erhitzungsprozess nahe der Siedetemperatur unterzogen wird.

Die Aussäuerung erfolgt vorzugsweise bis zu einem pH Wert von 3,3-3,5.
Gegenstand der Erfindung ist ein Heil- und Pflegemittel für die Haut auf Basis eines ausgesäuerten Sauerteiges erhältlich nach dem erfindungsgemäßen Verfahren.

Das erfindungsgemäße Heil- und Pflegemittel weist vorteilhafterweise einen hohen Anteil auf fermentativem Wege gewonnener Milchsäure von > 60 Gew.- % (als Anteil der Gesamtsäuremenge) auf.

Die erfindungsgemäßen Heil- und Pflegemittel sind mit in der Kosmetik üblichen Hilfs- und Trägerstoffen versetzbar, um Kosmetika herzustellen.

Die einsetzbaren erfindungsgemäßen Heil- und Pflegemittel werden insbesondere in Form von Pulvern, Roggenfluiden, Roggenseren, Roggengelen verwendet.

Das aus den erfindungsgemäßen Heil- und Pflegemittel herstellbare erfindungsgemäße Kosmetikum kann insbesondere als Wirkstoffzugabe von 1 bis 100 Gew.%, insbesondere 2 bis 50 Gew.% bezogen auf die Gesamtmenge in Salben, Pulvern, Cremes, Lotionen, Gelen, Shampoos, Haarspülungen, Packungen, Lippenstiften, Gesichts- und Körpermasken, oder Kombinationen davon eingesetzt werden.

So kann beispielsweise eine Gesichtsmaske zur Reinigung hergestellt werden aus in kosmetischen Rezepturen geläufigen Inkredenzien, wie Steareth-12 oder Steareth-21, Glycerylstearaten, Stearinsäure, Alkylbenzoaten mit Kettenlängen von insbesondere 12 bis 15 Kohlenstoffatomen, Cetearylisononanoat, Stearylheptanuat, Octylstearat, Duft- oder Aromastoffen, wie beispielsweise Persea Gratissima Avocadol (Firma CosmoCare), dem erfindungsgemäßen Roggengel oder Roggenfluid, Propyleneglycol, Füllstoffen wie Kaolin und Wasser. Eine andere Anwendungsform kann bestehen aus überwiegend erfindungsgemäßem Serum mit hohem Milchsäuregehalt sowie Vitaminen und anderen Ergänzungsstoffen. Ein Shampoo kann typischerweise Inhaltsstoffe enthalten, wie Sodiumlaurethsulfat, Dinatriumlaurethsulfosuccinat, Cocoamidopropylbetain, PEG-7 Glycerylcocoate, des erfindungsgemäße Roggenserum, Füllstoffe wie Styrolacrylatcopolymeren/Laureth-23, Zitronensäure, Polyquaternium-10 (Firma Amerchol), PEG-18 Glyceryloleat oder -cocoate sowie Wasser, Konservierungsstoffe und Duftstoffe. Eine typische Kurpackung kann bestehen aus Cetearylalkohol, Ceteareth-12, Ceteareth-20, PEG-75 Lanolin, Triticum Vulgare (Firma Dragoco), dem erfindungsgemäßen Roggenfluid oder Roggenserum, Hydroxycetyl oder Hedroxyethyl Dimonium Chlorith (Firma Henkel), Panthenol, Zitronensäure sowie Wasser, Konservierungsstoffen und Duftstoffen.

Ein Haarkonditionierer kann insbesondere bestehen aus dem erfindungsgemäßen Roggenfluid oder Roggenserum mit Duft-, Konservierungs- und Füllstoffen, beispielsweise Obstessig, Glycerin, Cetearylalkohol, Ceteareth-12, Cetrimoniumchlorith, PEG-75 Lanolin, Konservierungsstoffen, Duftstoffen sowie Wasser. Die genannten Inhaltsstoffe sind in der Kosmetik üblicherweise eingesetzte Chemikalien und sämtlich kommerziell erhältlich.

In einer Ausführungsform der vorliegenden Erfindung wird das erfindungsgemäße Heil- und Pflegemittel in Form eines Roggenfluids eingesetzt. Dabei handelt es sich um einen cremigen aus Roggengel gewonnenen Rohstoff, der sich auszeichnet in Cremes, Lotionen, Shampoos, Haarspülungen und Packungen.

Das erfindungsgemäße Roggengel ist ein eher dickflüssiger Rohstoff, der auch direkt als erfindungsgemäßes Heil- und Pflegemittel eingesetzt werden kann. Es handelt sich dabei um das mehr oder weniger unmittelbar aus dem Fermentationsprozess gewonnene Produkt, was sich in unveränderter Form als natürliche Gesichts- oder Körpermaske sowie als Badezusatz einsetzen lässt. Vorteilhaft an dieser Ausführungsform ist das Fehlen von den Organismus belastenden möglicherweise unverträglichen Bestandteilen. Nach Anwendung zeigen sich Verbesserungen des Hautreliefs und Verlangsamung der Bildung von Hautunreinheiten. Das Mittel kann als biologisches Peelingmittel abgestorbener Hautschuppen dienen, wobei vorteilhafterweise ein Moisturizing-Effekt in Erscheinung tritt.

Aus Roggenfluid kann seinerseits ein Roggenserum gewonnen werden. Es eignet sich als Rohstoff zum Einarbeiten in Gesichts- und Körpermasken, Lippenstiften, Haarshampoos und Haarspülungen sowie Haarpackungen.

Ausgehend vom Roggengel können beispielsweise Roggenpulver, Roggenfluid sowie Roggenserum hergestellt werden. Ausgehend von Roggengel kann das Roggenpulver hergestellt werden durch Separieren der Feststoffe aus dem erfindungsgemäßen Roggengel, das anschließend getrocknet und vermahlen wird. Die Feststoffe des Roggenpulvers können als Peelingkörper in den erfindungsgemäßen Kosmetika eingesetzt werden. Das Roggenfluid wird beispielsweise hergestellt durch Abtrennung vom gereinigten Gel und Anreicherung mit unfiltriertem Roggenserum bis die für die Verwendung geeignete Viskosität eingestellt ist. Es handelt sich bei diesem Produkt um einen Rohstoff mit hohem Wirkstoffanteil. Das Roggenserum wird beispielsweise hergestellt durch Filtrieren des flüssigen Überstandes des aus der Fermentation gewonnenen erfindungsgemäßen Roggengels und anschließende Klärung. Dabei entsteht ein Produkt mit sehr hohem Wirkstoffanteil.

Gegenstand der Erfindung ist auch die Verwendung der erfindungsgemäßen Heil- und Pflegemittel in einem Kosmetikum gemäß der Erfindung.

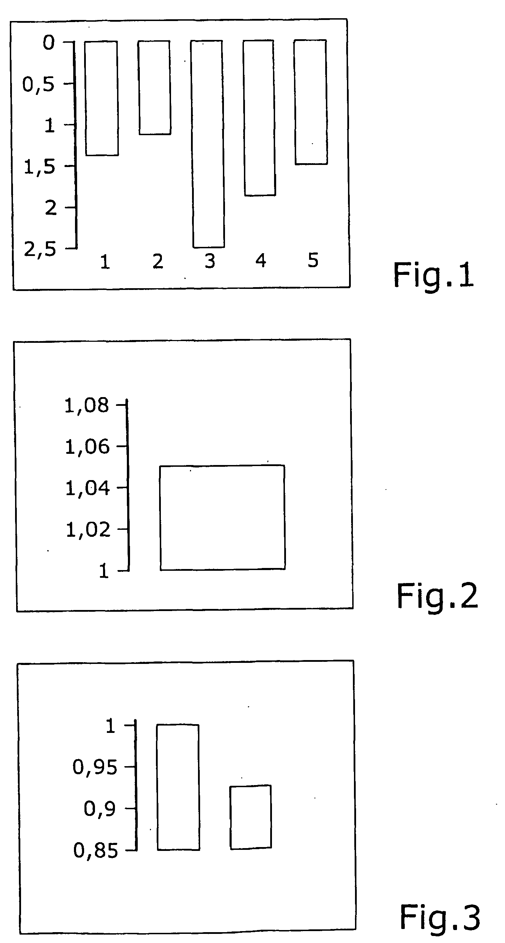

Fig. 1 zeigt die Beeinflussung des Erscheinungsbildes unreiner Haut durch die Behandlung mit einer Roggengelmaske. Die Reduzierung der Läsionen bei den Probanden 1 - 5 erfolgte um den Faktor bis zu 2,5.
Fig. 2 verdeutlicht den Moisturizing-Effekt. Es wird die Erhöhung der Hautfeuchtigkeit dargestellt.
Fig. 3 betrifft die Hautmikrotopographie und verdeutlicht die Reduktion der Haurauhigkeit bei zwei Probanden.

### Beispiel 1

Zur Herstellung des erfindungsgemäßen Roggengels wird ein Teil Roggenmehl 1150 mit zwei Teilen Wasser vermischt und einer Starterkultur mit α-Amylase versetzt. Die Mischung wird für 3 bis 5 Stunden auf 65 bis 75 °C erhitzt. Nach dem Start der enzymatischen Reaktion wird die Startfermentation eingeleitet durch Mischen von einem Teil Roggenmehl 1150 mit zwei Teilen Wasser. Dieser Mischung werden Fermentationsmedien mit Lactobacillus DSM 6037 sowie Lactobacillus DSM 6129 zugegeben. Diese Mischung wird für 24 bis 48 Stunden bei Temperaturen von 30 bis 34 °C fermentiert. Die Fermentation ist beendet, wenn sich ein konstanter pH-Wert einstellt. Die aus der Startfermentation gewonnene Mischung wird mit 60 Teilen, bezogen auf das Gesamtvolumen der Startfermentation, mit Wasser aufgefüllt. Die Fermentationszeit beträgt 24 bis 48 Stunden bis ein konstanter pH-Wert erreicht wird bei einer typischen Temperatur von 30 bis 34 °C. Danach erfolgt eine thermische Behandlung für 3 bis 5 Stunden bei einer Temperatur von 92 bis 100 °C. Gegebenenfalls werden Konservierungsmittel, wie Sorbinsäure und Benzoesäure, hinzugegeben. Typischerweise können 0,75 Teile Sorbinsäure bezogen auf das Gesamtvolumen eingesetzt werden. An den Konservierungsschritt schließen sich die Trennverfahren zur Herstellung der einzelnen Fraktionen, wie Roggenserum, Roggenfluid oder Roggenpulver an. Fraktionen von Roggenfluid und Roggenserum werden durch Zentrifugieren erhalten, wobei eine gute Trennung durch Hochleistungszentrifugen erfolgen muss. Deshalb werden unterschiedliche Zentrifugen eingesetzt sowie eine zusätzliche Filterung um eine hohe Reinheit zu garantieren. Das Roggenpulver wird durch schonende Trocknung erhalten.

### Beispiel 2

Im folgenden wird im einzelnen auf einige Untersuchungen eingegangen. Veränderungen der Anzahl der Auffälligkeiten in Relation zur unbehandelten Situation und zur Ausgangssituation.

### Anzahl der Komedonen und entzündlichen Läsionen

Die Behandlung mit dem Prüfmuster führte bei allen Probanden zu allen Meßzeitpunkten zur Reduktion der relativen Anzahl der entzündlichen Läsionen und Komedonen (Ausnahme Proband 1, Meßzeitpunkt 2 Wochen). Dabei sind Arealunterschiede und Veränderungen, die auf dem unbehandelten Areal z.B. durch klimatische Veränderungen oder individuelle Einflüssse zu verzeichnen waren, berücksichtigt. Unter Betrachtung aller Meßzeitpunkte unabhängig voneinander (Notwendigkeit zur Durchführung einer statistischen Betrachtung in Anbetracht der geringen Fallzahl) ergibt sich eine statistisch signifikante Reduktion (p < 0,05) sowohl der Anzahl der entzündlichen Läsionen als auch der Anzahl der Komedonen. Der Grad der Verbesserung der entzündlichen Läsionen und Komedonen ist relativ hoch. Eine Tendenz hinsichtlich der Dauer der Anwendung ist nicht erkennbar.

### Visuelle Beurteilung des Erscheinungsbildes der Haut

Das visuelle Erscheinungsbild der behandelten Gesichtshälfte stellte sich zu allen Meßzeitpunkten und bei allen Probanden leicht verbessert gegenüber der unbehandelten Gesichtshälfte dar. Der optische Unterschied betraf vorwiegend den Grad der Rötung und den Gesamteindruck sowie die Größe der einzelnen Auffälligkeiten.

### Subjektive Beurteilung durch den Probanden

Alle Probanden beurteilten die Wirkung des Prüfmusters als leicht bis eindeutig positiv. In 2 Fällen beschrieben die Probanden eine Verringerung der Hautrötung. 4 Probanden empfanden die behandelte Haut glatter als die unbehandelte. Eine Probandin beschrieb das Produkt als hautberuhigend.
Während der Anwendungphase empfanden 3 Probanden unmittelbar nach Produktauftrag eine negative sensuelle Empfindung in Form eines Spannens, leichten Prickeins bzw. Juckens.

### Diskussion

Das Prüfmuster Roggen-Gel bewirkte innerhalb des Prüfzeitraumes eine eindeutige Verbesserung des Hautzustandes unreiner Haut, wobei kein vollständiges Abklingen des Eindruckes der unreinen Haut beobachtet werden konnte. Die Probanden beschrieben eine positive Wirkung des Prüfmusters.

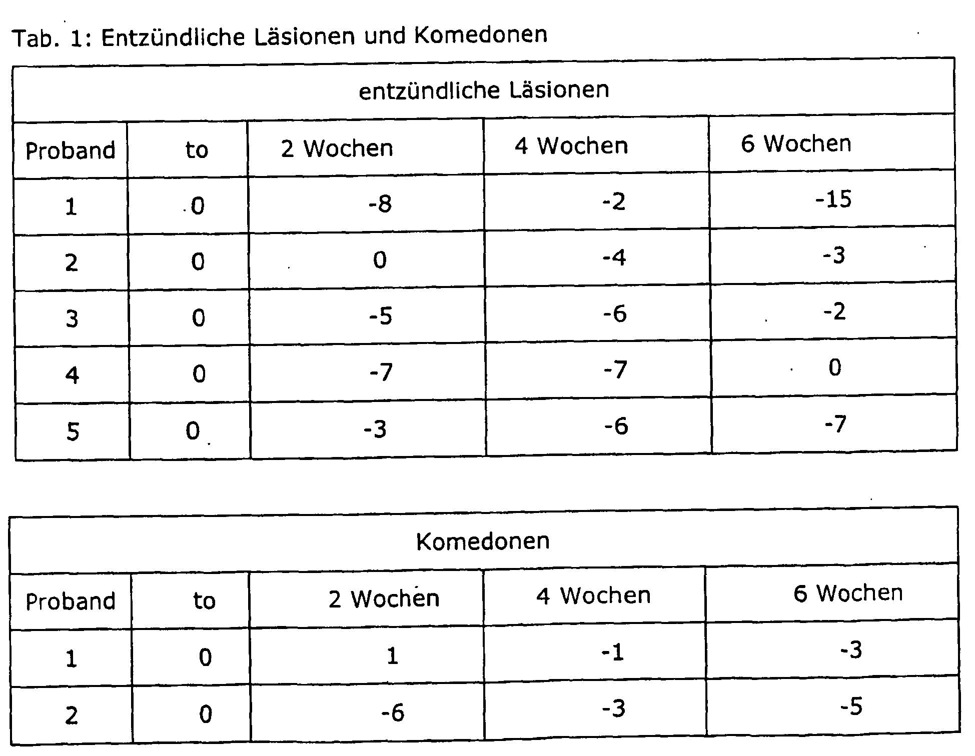

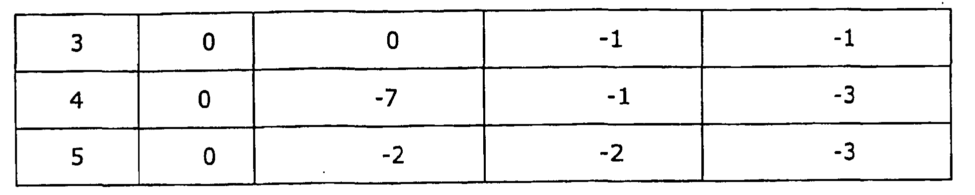

**Tab. 2: Randomisierung der Prüfmuster und Probandenspezifikation**

| Prob. | Geschlecht | Alter (y) | Areal (Gesichtshälfte) | |
|---|---|---|---|---|
| | | | links | rechts |
| 1 | w | 23,81 | behandelt | unbehandelt |
| 2 | m | 21,56 | unbehandelt | behandelt |
| 3 | m | 23,21 | behandelt | unbehandelt |
| 4 | w | 22,5 | unbehandelt | behandelt |
| 5 | m | 19,54 | behandelt | unbehandelt |

### Biometrie

### Zielgrößen

Die Zielgrößen der Hautpflegeprüfung sind die Hautfeuchtigkeit (Corneometrie) und die Hautmikrotopographie (Replika, R, DIN).

### Auswertung der Zielgrößen

Die Auswertung der Zielgrößen erfolgte durch Relativierung der Originaldaten der mit dem Prüfmuster behandelten Areale auf die unbehandelte Situation und den jeweiligen Ausgangswert (to). Es wurde der Mittelwert und die Standardabweichung der relativierten Daten bestimmt. Die Prüfung der Signifikanz von Unterschieden zwischen der unbehandelten und der behandelten Situation erfolgte mit der Software "STATISTICA". Zunächst wurde die Verteilung der Paardifferenzen der entsprechenden Paarvergleiche geprüft. Bei Normalverteilung der Paardifferenzen erfolgte die Signifikanzprüfung mittels zweiseitigem T-Test für abhängige Stichproben. Bei NichtNormalverteilung der Paardifferenzen wäre die Signifikanzprüfung mit dem Wilcoxon-Test für abhängige Stichproben erfolgt.

### Größe des Probandenkollektivs

Die Größe des Probandenkollektivs belief sich auf 15 ± 2.

### Diskussion

Es wurden die auf die unbehandelte Situation und den Ausgangszustand doppelt relativierten Daten ausgewertet, da diese die Veränderung der unbehandelten Kontrolle und die Unterschiede in der Ausgangssituation zwischen den unbehandelten und den behandelten Arealen berücksichtigen. Anhand der doppelt relativierten Daten zeichnet sich nach einer 7-tägigen Prüfmusterbehandlung eine leichte, aber signifikante Erhöhung der Hautfeuchtigkeit(um ca. 6 %) und eine leichte, aber signifikante Verringerung der Rauhigkeit (um ca. 7 %) gegenüber der unbehandelten Situation und der Ausgangssituation ab.

Signifikante Veränderungen hinsichtlich der Veränderung der Hautfeuchtigkeit und der Hautrauhigkeit konnten nach einer 21-tägigen Behandlung und der einwöchigen Behandlungspause nicht festgestellt werden. Das Prüfmuster Roggengel weist nur einen geringen befeuchtenden und glättenden Effekt auf die Haut auf.

Die klimatischen Verhältnisse im Prüfzeitraum spiegeln eine warme Sommerwetterlage mit Tagestemperaturen von ca. 16 °C bis 28 °C, viel Sonnenschein und einer relativ geringen Luftfeuchtigkeit wieder. Die erste Anwendungswoche war im Vergleich zu den anderen etwas kühler, bei geringerem Sonnenschein und einer etwas erhöhten Luftfeuchtigkeit. Die klimatischen Bedingungen an den Prüftagen waren unterschiedlich. Die Tagestemperaturen waren annähernd gleich, die Luftfeuchtigkeit schwankte um ca. 25 %. Die Meßklimabedingungen blieben während der 3 Prüftage konstant.

Aufgrund der Randomisierung der Prüfmuster starteten alle Prüfmuster mit den gleichen Ausgangsbedingungen.

Überprüfung der Einschlußkriterien Alle Probanden erfüllten die Einschlußkriterien.

### Meßergebnisse

### Hautfeuchtigkeit

Die Ausgangssituation stellt sich homogen dar, d.h. die unbehandelten und die zu behandelnden Areale weisen keine signifikanten Unterschiede hinsichtlich der Hautfeuchte auf.

Zum Zeitpunkt t₁ erhöhte sich durch die Prüfmusterbehandlung die Hautfeuchtigkeit im Mittel um ca. 6 % (± 12 %) gegenüber der unbehandelten Situation und dem Ausgangszustand. Dieser Unterschied ist signifikant. Nach der 3-wöchigen Behandlung ist im Mittel nur noch eine sehr leichte, aber nicht signifikante Erhöhung der Hautfeuchtemeßwerte durch die Prüfmusterbehandlung zu verzeichnen (ca. 2 %). Nach der einwöchigen Behandlungspause ist im Mittel die Hautfeuchte leicht, aber nicht signifikant erhöht gegenüber der unbehandelten Situation und dem Ausgangswert (ca. 5 %)

### Hautmikrotopographie

Die Originaldaten der Bestimmung der Hautrauhigkeit (Replika, R_{z} DIN) sind im Anhang unter, die auf die unbehandelte Situation und to relativierten Daten unter dargestellt.

Die Ausgangssituation ist nicht homogen, d.h. die unbehandelten Areale sind signifikant glatter als die zu behandelnden Hautareale.

Zum Zeitpunkt t₁ verringerte sich durch die Prüfmusterbehandlung die Rauhigkeit im Mittel um ca. 7,5 % (± 7 %) gegenüber der unbehandelten Situation und dem Ausgangszustand. Dieser Unterschied ist signifikant. Nach der 3-wöchigen Behandlung und der einwöchigen Behandlungspause ist im Mittel keine Verringerung der Rauheitsmeßwerte durch die Prüfmusterbehandlung mehr zu verzeichnen.

### Beispiel 3

Im folgenden wird die Analyse des erfindungsgemäßen Heil- und Pflegemittels angegeben.

Bei Voruntersuchungen zur Auftrennung einer Sauerteig-Wasser-Suspension mittels Zentrifugalsedimentation wurde ein 3-Phasen-System festgestellt.

Im Rahmen dieser Untersuchung sollte festgestellt werden, ob es sich bei der Mittelphase um eine stabile handelt, woraus sie aufgebaut sein könnte und ob es dort gegenüber des wäßrigen Überstandes zu einer Anreicherung wichtiger Inhaltsstoffe wie organische Säuren, Proteine und wasserlösliche Vitamine kommt. Auslegungshinweise für eine Separierung der Feststoffe sollten erlangt werden.

### Methoden

### Zentrifugation der Sauerteig-Suspension

Verwendete Geräte:
Tischzentrifuge Hermie z 320
Laborzentrifuge: Hermie z 513
Rheometrische Untersuchungen der abgetrennten Phasen
Verwendetes Gerät: Rheometer: carri med csl (controi stress rheometer)
Methode: Fließkurvenermittlung mit vorgegebener Schubspannung und gemessener Schergeschwindigkeit bei einer Temperatur von 23 °C.

### Chemische Analysen

Probengewinnung: Um genügend Probe für die chemischen Analysen zu erhalten wurden 1600 g Sauerteigmischung bei 4300 Upm für 40 Minuten zentrifugiert. Die einzelnen Phasen wurden sorgfältig abpipettiert und für die externe Analyse eingefroren.

Alle chemischen Analysen erfolgten am Deutschen Institut für Lebensmitteltechnik. Methoden siehe Ergebnisse.

**Tab. 3: Analysenergebnisse des klaren, wäßrigen Überstandes**

| Parameter | Verfahren | Einheit | Meßwert |
|---|---|---|---|
| Rohprotein | Kjeldahl | g/100 g | 0,73 |
| Essigsäure | HPLC-Refr. | G/100 g | 0,06 |
| Milchsäure | HPLC-Refr. | G/100 g | 0,64 |
| Propionsäure | HPLC-Refr. | G/100 g | <0,01 |
| Buttersäure | HPLC-Refr. | G/100 g | <0,01 |
| Ascorbin/Dehydroascorbinsäure | HPLC- Fluoresz | mg/100 g | <5 |
| Vitamin B 2 | HPL-J-UVD | mg/100 g | < 0,5 |
| Vitamin B 6 (Pyridoxin) | HFL-C-UVD | mg/100 g | < 1 |
| Folsäure | HPLC-UVD | mg/100 g | < 2 |
| Ethanol | HPLC-Refr. | g/100.g | 0,07 |

**Tab. 4: Analysenergebnisse des milchigen, gelartigen Überstandes (Mittelphase)**

| Parameter | Verfahren | Einheit | Meßwert |
|---|---|---|---|
| Rohprotein | Kjeldahl | g/100 g | 0,83 |
| Essigsäure | HPLC-Refr. | g/100 g | 0,05 |
| Milchsäure | HPLC-Refr. | g/100 g | 0,64 |
| Propionsäure | HPLC-Refr. | g/100 g | < 0,01 |

| Buttersäure | HPLC-Refr.. | g/100 g | < 0,01 |
|---|---|---|---|
| Ascorbin-/Dehydroascorbinsäure | HPLC- Fluoresz. | Mg/100 g | < 5 |
| Vitamin B₂ | HPLC-UVD | mg/100 g | < 0,5 |
| Vitamin B₆ (Pyridoxin) | HPLC-UVD | mg/100 g | < 1 |
| Folsäure | HPLC-UVD | mg/100 g | < 2 |
| Ethanol | HPLC-Refr. | g/100 g | 0,07 |

### Beispiel 4

Das erfindungsgemäße Kosmetikmittel ist geeignet bei folgenden Erscheinungen eingesetzt zu werden.

### AKNE:

Fermentierter Rohstoff wurde in der Anfangsphase frisch (lebende Kulturen) auf die Gesichtshaut aufgetragen und nach abtrocknen der "Maske" mit warmem Wasser abgenommen.

Als Alternative kann auch eine pasteurisierte "Maske" verwendet werden.

Die Ergebnisse wurden von den Probanden, die beide Produkte angewendet haben, als nahezu identisch bewertet:

Rosige, gut durchblutete, weiche Haut, Aknebild war stark verbessert. In zwei Fällen wurde ein totales Abheilen beobachtet. Nach 3 Tagen setzte allerdings erneut die Bildung von Aknepusteln ein, die dann aber wieder mit einer erfindungsgemäßen Ferment-Regenerationsmaske behandelt werden konnten.

Bei Aknenarben konnten nach drei Behandlungen schon wesentliche Verbesserungen gestellt werden. Die Haut sah glatter aus und wirkte gepflegt und gut durchblutet (Behandlung siehe oben).

### KOPFHAUT:

Hier hat sich das erfindungsgemäße Kosmetikmittel in Form des Serums bei verschiedenen Problemen bewährt.

Eine typische Anwendung ist wie folgt:

Vor dem Waschen wird das Haar gescheitelt und das Produkt Strich für Strich einmassiert. Nach einer Einwirkzeit von 15 Minuten wäscht man mit einem milden Shampoo das Haar wie gewohnt.

Schuppen, entzündliche Partien, bis hin zu partiellem Haarausfall nach Ablösen größerer Kopfhautzonen konnten verbessert und um Teil sogar geheilt werden.

### Ergebnisse

### Abweichungen vom Prüfplan

Es traten keine Abweichungen vom Prüfplan auf.

### Unerwünschte Ereignisse 1 Ausfälle

Es traten keine unvorhergesehenen Ereignisse auf. 5 Probanden absolvierten die Prüfung korrekt und vollständig.

### Überprüfung der Einschlußkriterien

Alle Probanden erfüllten die Einschlußkriterien.

### Applikationszeiten der Prüfmuster

Die Applikationszeiten der Prüfmuster entsprachen den Anforderungen.

### Ergebnisse der visuellen Begutachtung

Es wurde die Verträglichkeit von 6 Prüfmustern, jeweils unverdünnt und in der Verdünnung 50 % anhand einer einmaligen Applikation der Prüfmuster untersucht. Dabei wurde ein Prüfmuster als auffällig bewertet, wenn das Ergebnis der visuellen Bewertung ≥ 1,0 lautete, d.h. mindestens eine leichte, punktförmige oder diffuse Rötung auftrat.

Die Prüfmuster Weizen-Serum, Weizen-Gel, Hafer-Gel (unverdünnt und 50 %) und die Prüfmuster Hafer-Serum und Roggen-Gel (unverdünnt) waren bei keinem Probanden auffällig.

Auf die Prüfmuster Hafer-Serum und Roggen-Gel in der Verdünnung 50 % und das Prüfmuster Roggen-Serum (unverdünnt und 50 %) reagierte jeweils 1 Proband (20 % aller Probanden) mit einer leichten punktförmigen oder diffusen Rötung. Das Maximum der Reaktion auf diese Prüfmuster trat 24 h nach Expositionsende auf. Die Reaktionen waren in allen Fällen auf das Applikationsareal begrenzt.

## Patentansprüche

1. Verfahren zur Herstellung eines Heil- und Pflegemittels für die Haut auf Basis eines ausgesäuerten Sauerteiges, wobei
1.1 einem Roggenmehl oder Roggenschrot ein natürlicher enzymatischer Starter und Wasser zugegeben wird, die Mischung einem Erhitzungsprozess auf 65 -75°C innerhalb von 3-5 Stunden ausgesetzt wird, wobei durch enzymatische Reaktionen eine starke Maltosebildung einsetzt,
1.2. eine weitere Zugabe von Roggenmehl oder Roggenschrot, Wasser sowie eines bakteriologischen Impfguts aus der Gruppe der heterofermentativen Milchsäurebakterien, erfolgt
1.3. und der Ansatz bis zur Einstellung der Stoffwechseltätigkeit der Mikroorganismen ausgesäuert wird.

2. Verfahren nach Anspruch 1, wobei der enzymatische Starter α-Amylase ist.

3. Verfahren nach Anspruch 1 und/oder 2 wobei die heterofermentativen Milchsäurebakterien Lactobacillus DSM 6037 und/oder Lactobacillus DSM 6129 sind.

4. Verfahren nach einem der Ansprüche 1 bis 3, wobei dem Fermentationsprodukt ein zweites kohlehydrathaltiges Substrat und Wasser sowie ein bakteriologisches Impfgut aus der Gruppe der heterofermentativen Milchsäurebakterien zugegeben und die Mischung ausgesäuert wird,
- der letzte Verfahrensschritt ggf. ein oder mehrmals wiederholt wird, wobei die Mischung abschließend bis zur Einstellung jeglicher bakteriologischer Stoffwechselaktivität ausgesäuert wird und
- das Fermentationsprodukt einem stabilisierenden Erhitzungsprozess nahe der Siedetemperatur unterzogen wird.

5. Heil- und Pflegemittel für die Haut auf Basis eines ausgesäuerten Sauerteiges erhältlich nach dem Verfahren nach einem der Ansprüche 1 bis 4.

6. Heil- und Pflegemittel nach Anspruch 5, **dadurch gekennzeichnet, dass** das Heil- und Pflegemittel einen hohen Anteil auf fermentativem Wege gewonnener Milchsäure von > 60 % als Anteil der Gesamtsäuremenge aufweist, wobei vor der Aussäuerung des Sauerteigs eine Behandlung mit α-Amylase erfolgt.

7. Heil- und Pflegemittel nach Anspruch 5 und/oder 6 mit in der Kosmetik üblichen Hilfs- und Trägerstoffen.

8. Heil- und Pflegemittel nach mindestens einem der Ansprüche 5 bis 7 in Form von Pulvern, Roggenfluiden, Roggenseren, Roggengelen.

9. Kosmetikum enthaltend ein Heil- und Pflegemittel nach mindestens einem der Ansprüche 5 bis 8, in Form von Salben, Cremes, Lotionen, Gelen, Shampoos, Haarspülungen, Packungen, Lippenstiften, Gesichts- und Körpermasken, oder Kombinationen davon.

10. Verwendung von Heil- und Pflegemittel nach mindestens einem der Ansprüche 5 bis 8 in einem Kosmetikum nach Anspruch 9.

## Claims

1. A method for the preparation of a curative and care agent for the skin on the basis of an acidified leaven, wherein:
1.1 a natural enzymatic starter and water are added to a rye fine or coarse meal, the mixture is subjected to a process of heating to 65-75 °C within 3 to 5 hours, a strong maltose formation being initiated from enzymatic reactions;
1.2 a further addition of rye fine or coarse meal, water and a bacteriological inoculum from the group of heterofermentative lactic-acid bacteria is made; and
1.3 the mixture is acidified until the metabolic activity of the microorganisms ceases.

2. The method according to claim 1, wherein said enzymatic starter is α-amylase.

3. The method according to claim 1 and/or 2, wherein said heterofermentative lactic-acid bacteria are *Lactobacillus* DSM 6037 and/or *Lactobacillus* DSM 6129.

4. The method according to any of claims 1 to 3, wherein a second carbohydrate-containing substrate and water as well as a bacteriological inoculum from the group of heterofermentative lactic-acid bacteria are added to the fermentation product, and the mixture is acidified;
- the latter process step is optionally repeated once or several times, and the mixture is finally acidified until any bacteriological metabolic activity ceases; and
- the fermentation product is subjected to a stabilizing heating process at near to the boiling temperature.

5. A curative and care agent for the skin on the basis of an acidified leaven obtainable by the method according to any of claims 1 to 4.

6. The curative and care agent according to claim 5, **characterized in that** said curative and care agent has a high content of lactic acid obtained in a fermentative way of > 60% as a fraction of the total amount of acid , wherein a treatment with α-amylase is performed prior to acidification of the leaven.

7. The curative and care agent according to claim 5 and/or 6 comprising auxiliary agents and carriers usual in the cosmetic art.

8. The curative and care agent according to at least one of claims 5 to 7 in the form of powders, rye fluids, rye sera, rye gels.

9. A cosmetic preparation containing a curative and care agent according to at least one of claims 5 to 8 in the form of ointments, creams, lotions, gels, shampoos, hair conditioners, packs, lipsticks, face and body masks, or combinations thereof.

10. Use of a curative and care agent according to at least one of claims 5 to 8 in a cosmetic preparation according to claim 9.

## Revendications

1. Procédé de fabrication d'un produit curatif et de soin pour la peau à base de levain traité à l'acide,
1.1 une amorce enzymatique naturelle et de l'eau étant ajoutées à une farine de seigle ou à un gruau de seigle, le mélange étant exposé à un processus de chauffage à 65-75 °C dans un intervalle de 3 à 5 heures, une formation de maltose importante se mettant en place par des réactions enzymatiques,
1.2 un autre ajout de farine de seigle ou de gruau de seigle, d'eau ainsi que d'un inoculum bactériologique du groupe des bactéries lactiques hétérofermentatives étant effectué,
1.3 et la charge de départ étant traitée à l'acide jusqu'à cessation de l'activité métabolique des microorganismes.

2. Procédé selon la revendication 1, l'amorce enzymatique étant l'α-amylase.

3. Procédé selon la revendication 1 et/ou 2, les bactéries lactiques hétérofermentatives étant le lactobacille DSM 6037 et/ou le lactobacille DSM 6129.

4. Procédé selon l'une des revendications 1 à 3, dans lequel un second substrat glucidique et de l'eau ainsi qu'un inoculum bactériologique du groupe des bactéries lactiques hétérofermentatives sont ajoutés et le mélange est traité à l'acide,
- la dernière étape du procédé est, le cas échéant, répétée une ou plusieurs fois, le mélange étant par la suite traité à l'acide jusqu'à cessation de chaque activité métabolique bactériologique et
- le produit de fermentation est soumis à un processus de chauffage stabilisateur proche de la température d'ébullition.

5. Produit curatif et de soin pour la peau à base de levain traité à l'acide pouvant être obtenu avec le procédé selon l'une des revendications 1 à 4.

6. Produit curatif et de soin selon la revendication 5, **caractérisé en ce que** le produit curatif et de soin présente une proportion élevée d'acide lactique obtenu par fermentation supérieure à 60 % en tant que proportion de la quantité d'acide totale, un traitement avec l'α-amylase étant effectué avant le traitement à l'acide du levain.

7. Produit curatif et de soin selon la revendication 5 et/ou 6 avec des auxiliaires et des véhicules habituels en cosmétique.

8. Produit curatif et de soin selon au moins l'une des revendications 5 à 7, sous forme de poudres, de fluides à base de seigle, de sérums à base de seigle et de gels à base de seigle.

9. Produit cosmétique contenant un produit curatif et de soin selon au moins l'une des revendications 5 à 8, sous forme de pommades, de crèmes, de lotions, de gels, de shampooings, d'après-shampooings, d'enveloppements, de bâtons de rouge à lèvres, de masques pour le visage et le corps ou de combinaisons de ceux-ci.

10. Utilisation du produit curatif et de soin selon l'une des revendications 5 à 8 dans un produit cosmétique selon la revendication 9.
